# EUROPEAN PATENT SPECIFICATION

(11) **EP 4 501 268 B1**
(45) Date of publication and mention of the grant of the patent: **18.03.2026**
(21) Application number: 22942700.0
(22) Date of filing: 19.05.2022
(51) Int. Cl.: A61B 34/00, A61B 34/30, A61B 90/70, A61B 90/00

(54) **SURGICAL INSTRUMENT**
CHIRURGISCHES INSTRUMENT
INSTRUMENT CHIRURGICAL

(43) Date of publication of application: 05.02.2025
(73) Proprietor: RIVERFIELD Inc., 1070052 Tokyo (JP)
(72) Inventor: SHINDO, Koki, Tokyo 107-0052 (JP); TAKIKAWA, Kyohei, Tokyo 107-0052 (JP)
(74) Representative: Adares PartGmbB
(86) International application number: PCT/JP2022/020810
(87) International publication number: WO 2023/223495

(56) References cited:
- CN-A- 112 603 547
- JP-A- 2009 028 156
- JP-A- 2009 028 156
- JP-A- 2010 207 260
- JP-A- 2010 207 260
- US-A1- 2013 345 701
- US-A1- 2013 345 701
- US-A1- 2016 193 012
- US-A1- 2017 128 697
- US-A1- 2021 007 828
- US-A1- 2021 369 400

## Description

### TECHNICAL FIELD

The present disclosure relates to a surgical instrument.

### BACKGROUND ART

A surgical instrument that includes a cartridge having an inner space; a cylindrical shaft extending from the cartridge; and an end effector, such as a forceps, disposed at a leading end of the cylindrical shaft is known. The surgical instrument is cleaned and sterilized before or after use.

As a method of cleaning the surgical instrument, a method that uses a cleaning solution is known. For example, a method of cleaning the surgical instrument by supplying the cleaning solution in an inside of the cartridge and in an inside of the shaft is known (for example, see Patent Document 1).

The cleaning solution is supplied through a flush port disposed in the cartridge and a main flush port which is different from the flush port. The cleaning solution supplied through the flush port is supplied to the inside of the cartridge. The inside of the cartridge is cleaned with the cleaning solution supplied from the flush port.

The cleaning solution supplied through the main flush port is supplied to the inside of the shaft via a tube extending from the main flush port. The tube is situated in the inside of the cartridge, from the main flush port to the inside of the shaft. The inside of the shaft is cleaned with the cleaning solution supplied through the main flush port and the tube.

Patent Document 2 describes an instrument including fluid ports to permit circulation of a cleaning solution through a housing. When the ports are opened, a cleaning solution or sterilization fluid can be circulated through the housing past interior components. An additional or alternative port can be disposed on a shaft member which extends from the housing.

Patent Document 3 describes a forceps comprising a shaft portion, a handle portion comprising handles, shaft portions, and a shaft. An operating area is surrounded by a first fixed handle case and a second fixed handle case and connected to a first opening and a second opening.

Patent Document 4 describes a medical manipulator comprising a connection portion main body, a connection shaft, an inner cavity having an opening and communicating with the connecting shaft, a first cleaning hole and a second cleaning hole.

Patent Document 5 describes a surgical instrument comprising a proximal portion, and a shaft. The surgical instrument can include an open architecture permitting cleaning fluid to flow within the proximal portion and within shaft to exit shaft, and a portion of the cleaning fluid can flow out of proximal portion.

Patent Document 6 describes a surgical instrument including a plurality of driven members, a housing, a cleaning tube, and a shaft. Further, it comprises a first cleaning liquid supply hole to which the cleaning tube is attached, a second cleaning liquid supply hole communicating with the inside of the housing, and a third cleaning liquid supply hole communicating with the inside of the housing. Cleaning liquid can be filled into one of the cleaning liquid supply holes and leaving the housing by gaps of the housing.

Patent Document 7 describes a robotic surgical tool autowasher system comprising a frame with a drive housing, a shoulder, a basin defining fluid apertures, internal conduits in fluid communication with fluid nozzles or outlets, and a fitting.

Patent Document 8 describes a surgical instrument comprising a driver, an end effector and a long shaft connected between the driver and the end effector, and a liquid inlet, and a flow guide structure in fluid communication with the liquid inlet.

### PRIOR ART DOCUMENTS

### PATENT DOCUMENTS

Patent Document 1: Japanese Unexamined Patent Application Publication No. 2021-186231
Patent Document 2: US 2013/345701 A1
Patent Document 3: JP 2010 207260 A
Patent Document 4: JP 2009 028156 A
Patent Document 5: US 2016/193012 A1
Patent Document 6: US 2021/369400 A1
Patent Document 7: US 2021/007828 A1
Patent Document 8: CN 112 603 547 A

### SUMMARY OF THE INVENTION

### PROBLEMS TO BE SOLVED BY THE INVENTION

Both the cartridge and the shaft have an opening where they are connected to each other. In other words, an inner space of the cartridge communicates with an inner space of the shaft. Most of the cleaning solution used to clean the inside of the cartridge is discharged to outside of the cartridge. Meanwhile, a part of the cleaning solution used to clean the inside of the cartridge flows from the inner space of the cartridge into the inner space of the shaft.

It is often the case that the shaft does not have an opening for the cleaning solution to flow to the outside. In most cases, the cleaning solution in the shaft flows to the outside from the shaft via the cartridge. Thus, it has been necessary to bring the cleaning solution that had entered the shaft from the cartridge after cleaning back to the cartridge to discharge the cleaning solution to the outside from the cartridge. Accordingly, the time required for cleaning the surgical instrument has tended to be long, and therefore, cleaning efficiency has tended to be poor. Hereinafter, the cartridge is also referred to as "housing" in the present disclosure.

The present disclosure discloses one example of a surgical instrument that easily inhibits a deterioration of the cleaning efficiency.

### MEANS FOR SOLVING THE PROBLEMS

The problem is solved by a surgical instrument according to claim 1. A surgical instrument which is one aspect of the present disclosure includes a housing including an inner space, and a shaft formed into a cylindrical shape extending from the housing and communicating with the inner space. The housing includes a flush port that is a through hole for a cleaning solution to enter from the outside, and at least one aperture from which the cleaning solution flows to the outside. The flush port is situated at a position that allows a center axis of the shaft to be interposed between the flush port and the at least one aperture.

According to the surgical instrument of the present disclosure, the cleaning solution that entered the inside of the housing from the flush port crosses the center axis of the shaft and is likely to flow to the outside of the housing from the at least one aperture. The direction in which the cleaning solution flows is a direction that crosses the center axis of the shaft. Among components of the direction of flow of the cleaning solution, a component of the flow towards the inside of the shaft is likely to be smaller than a component of the flow towards the at least one aperture. In other words, the direction of the flow of the cleaning solution is less likely to be directed from the inner space of the housing towards the inside of the shaft.

In the surgical instrument which is one aspect of the present disclosure, it is preferable that the flush port is situated such that an axis of the flush port extends through a position different from a position where the center axis of the shaft passes through.

Due to such a configuration, a main direction of the flow of the cleaning solution that enters from the flush port along the axis of the flush port is likely to be different from a direction towards the inside of the shaft. Thus, the cleaning solution supplied to the inside of the housing through the flush port is less likely to enter the inside of the shaft from the inner space of the housing. Hereinafter, the positional relationship between the flush port and the shaft, in which the axis of the flush port extends through a position different from a position through which the center axis of the shaft extends, is also referred to as a twisted positional relationship.

In the surgical instrument, the flush port is situated in an area of the housing closer to the shaft than the at least one aperture is.

Due to such a configuration, among the components of the direction of the flow of the cleaning solution that entered the inside of the housing from the flush port, a component of the negative direction of the Z-axis (a direction away from the shaft along the center axis of the shaft) is likely to increase. The cleaning solution is less likely to flow from the inner space of the housing to the inside of the shaft.

The surgical instrument includes the housing including the inner space, and the shaft formed into a cylindrical shape extending from the housing and communicating with the inner space. The housing includes the flush port that is a through hole for the cleaning solution to enter from the outside. The flush port is situated in an area in the housing closer to the shaft than to the center of the housing in a direction in which the shaft extends.

According to the surgical instrument of the present disclosure, a main direction of flow of the cleaning solution supplied from the flush port to the inside of the housing is likely to be directed to the center of the housing. In other words, the cleaning solution is likely to flow in a direction away from the shaft. The cleaning solution is less likely to flow from the inner space of the housing to the inside of the shaft.

In the surgical instrument which is one aspect of the present disclosure, it is preferable that the flush port is situated such that the axis of the flush port passes and extends through a position different from the position through which the center axis of the shaft extends.

Due to such a configuration, the main direction of flow of the cleaning solution from the flush port along the axis of the flush port is likely to be different from the direction towards the inside of the shaft. The cleaning solution is less likely to enter the inside of the shaft from the inner space of the housing.

In the surgical instrument, the housing includes at least one aperture through which the cleaning solution flows to the outside, and the at least one aperture is situated at a position that allows the center axis of the shaft to be interposed between the at least one aperture and the flush port.

Due to such a configuration, the main direction of flow of the cleaning solution is likely to be directed towards the at least one aperture Thus, the direction of flow of the cleaning solution is likely to cross the center axis of the shaft. Among the components of the direction of flow of the cleaning solution, a component of flow towards the inside of the shaft is likely to be smaller than components of flow towards the at least one aperture. In other words, the direction of flow of the cleaning solution is less likely to be directed from the inner space of the housing towards the inside of the shaft.

### EFFECTS OF THE INVENTION

According to the surgical instrument of the present disclosure, the flush port is situated at a position that allows the center axis of the shaft to be interposed between the flush port and the at least one aperture. Thus, the flow of the cleaning solution is less likely to be directed from the inner space of the housing towards the inside of the shaft, which yields an effect that a deterioration of cleaning efficiency is easily inhibited.

According to the surgical instrument of the present disclosure, the flush port is situated in an area of the housing closer to the shaft than to the center of the housing in a direction in which the shaft extends. Thus, the direction of flow of the cleaning solution is less likely to be directed from the inner space of the housing towards the inside of the shaft, which yields an effect that a deterioration of cleaning efficiency is easily inhibited

### BRIEF DESCRIPTION OF THE DRAWINGS

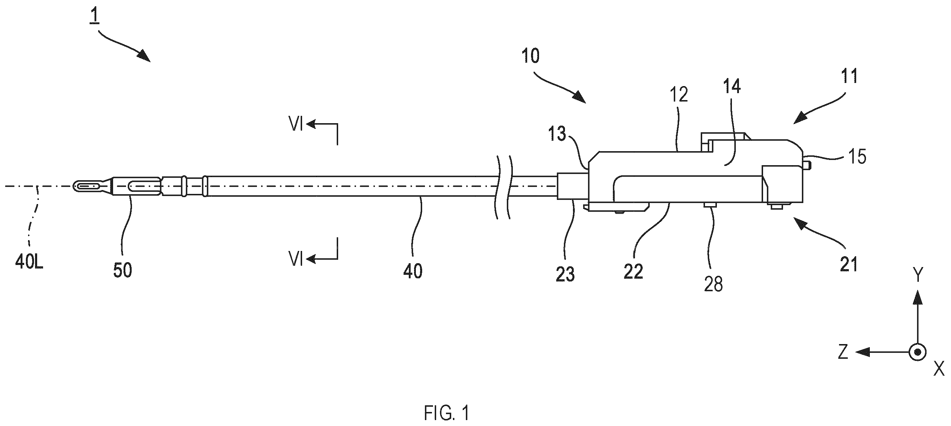

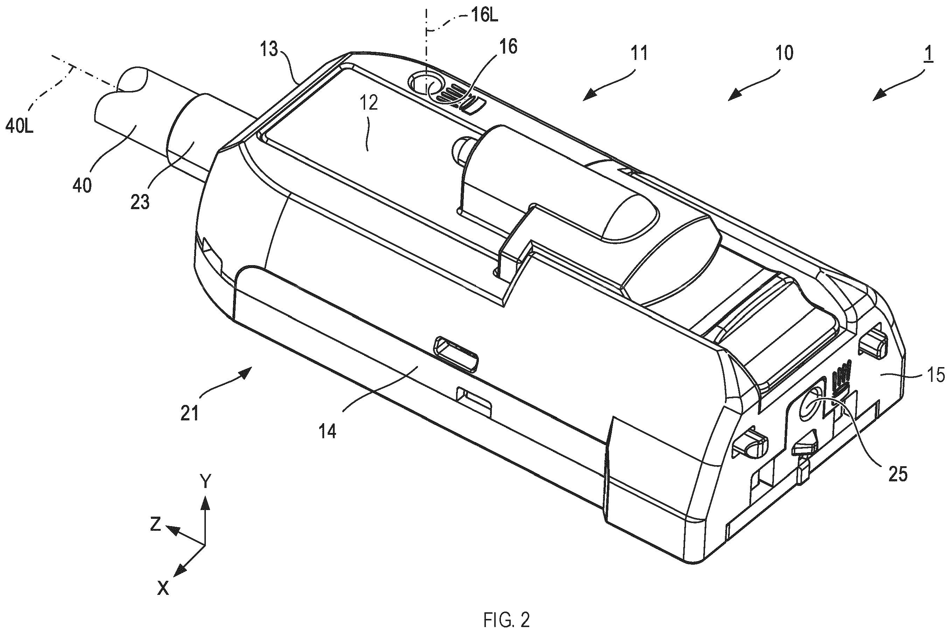

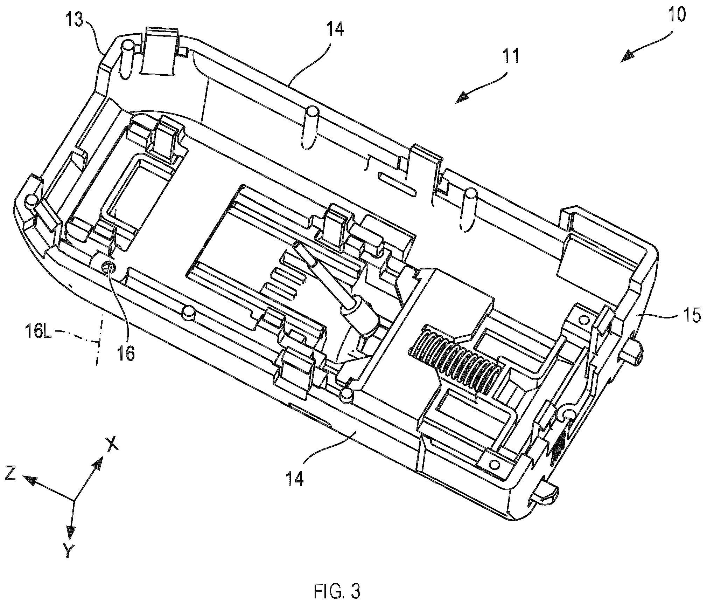

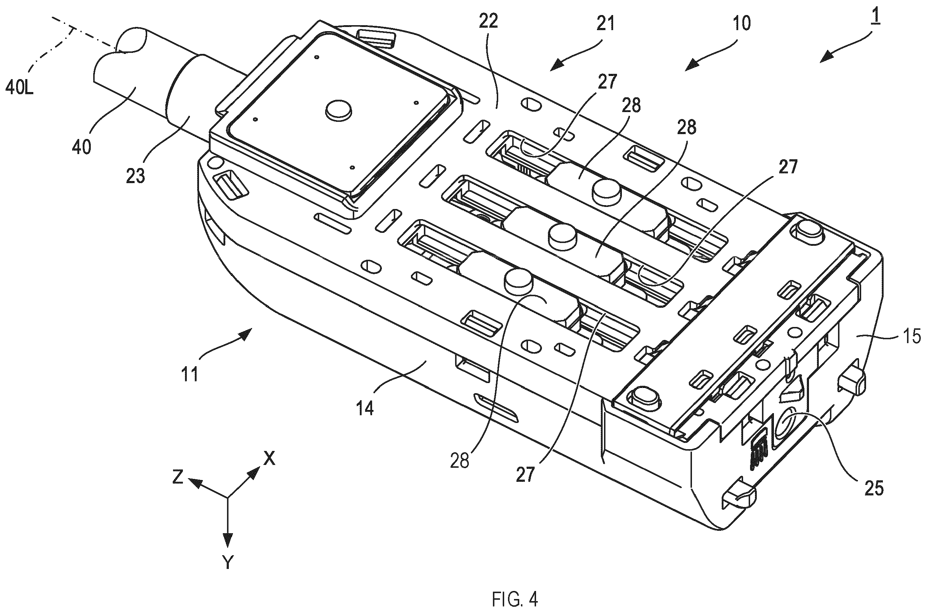

FIG. I is a front view describing a configuration of a surgical instrument of the present disclosure. FIG 2 is a perspective view describing an outer configuration of a housing and a cover of FIG. 1. FIG. 3 is a perspective view describing an inner configuration of the cover of FIG. 1. FIG. 4 is a perspective view describing an outer configuration of a base of FIG. 1.
FIG. 5 is a perspective view describing an inner configuration of the base of FIG. 1.
FIG. 6 is a cross-sectional view describing a configuration of an inside of a shaft of FIG. 1 taken along arrows VI-VI in FIG. 1.
FIG. 7 is a schematic diagram describing a flow of a cleaning solution in the surgical instrument.
FIG. 8 is a schematic diagram describing a flow of the cleaning solution in the housing.
FIG. 9 is a schematic diagram describing a flow of the cleaning solution in the shaft.
FIG. 10 is a schematic diagram describing a flow of a different cleaning solution in the surgical instrument.

### EXPLANATION OF REFERENCE NUMERALS

1... surgical instrument; 10...housing; 16...flush port; 16L...axis; 26... tube; 27... aperture; 40... shaft; 40L... center axis.

### MODE FOR CARRYING OUT THE INVENTION

A surgical instrument 1 according to an embodiment of the present disclosure will be explained with reference to FIG. 1 to FIG. 8. The surgical instrument 1 in the present embodiment is an instrument used in surgeries and attached to a master-slave-type surgical robot. The surgical instrument 1 may be attached to surgical robots other than the master-slave-type surgical robot. Furthermore, the surgical instrument 1 may also be an instrument that is not attached to a surgical robot. FIG. 1 to FIG. 8 do not illustrate a surgical robot.

The surgical instrument 1 according to the present embodiment is an instrument that is moved by a driving force transmitted from outside. The driving force is generated in an external drive source, such as an actuator, and is transmitted to the surgical instrument 1 via a transmitting body, such as a wire.

As shown in FIG. 1, the surgical instrument 1 includes a housing 10 and a shaft 40. The surgical instrument 1 further includes an end effector 50.

Hereinafter, explanations will be given by referring to an X-direction, a Y-direction, and a Z-direction which will be explained below. The X-direction and the Y-direction are directions extending along a plane situated perpendicular to a center axis 40L of the shaft 40 that extends along the surface of FIG. 1. The X-direction and the Y-direction are perpendicular to each other. The plane situated perpendicular to the center axis 40L of the shaft 40 is perpendicular to the surface of FIG. 1.

In other words, the X-direction is a direction extending along a mounting surface 22 of a base 21 of the housing 10. The positive direction of the X-direction is a direction that extends outward from the surface of FIG. 1. In other words, the positive direction of the Y-direction is a direction from the base 21 of the housing 10 towards a cover 11. The Z-direction is a direction parallel to the center axis 40L. The positive direction of the Z-direction is a direction from the housing 10 towards the end effector 50.

The housing 10 is a container having an inner space. The housing 10 is a part of the surgical instrument 1 that is attached to a surgical robot. As shown in FIG. 2, the housing 10 includes the cover 11 and the base 21.

The cover 11 is a member that forms an outer shape and the inner space of the housing 10 together with the base 21. As shown in FIG. 2 and FIG. 3, the cover 11 includes an upper surface 12, a front surface 13, lateral surfaces 14, 14, and a back surface 15.

The upper surface 12 is a surface extending along a plane Z-X and is situated at the positive side in the Y-direction in the cover 11. The front surface 13 is a surface extending along a plane X-Y and is situated at the positive side in the Z-direction in the cover 11. The lateral surfaces 14, 14 are surfaces extending along a plane Y-Z and is situated at the positive side and the negative side in the X-direction in the cover 11. The back surface 15 is a surface extending along a plane X-Y and is situated at the negative side in the Z-direction in the cover 11.

The upper surface 12, the front surface 13, the lateral surfaces 14,14, and the back surface 15 each may only include a flat surface, each may include a combination of a flat surface and a curved surface, or each may only include a curved surface. The upper surface 12, the front surface 13, the lateral surfaces 14, 14, and the back surface 15 each may also include a protrusion or a recess.

As shown in FIG. 2, the cover 11 includes a flush port 16. More specifically, the flush port 16 is disposed on the upper surface 12 of the cover 11. The flush port 16 may be disposed on the front surface 13 of the cover 11, or on the lateral surfaces 14, 14.

The flush port 16 is situated at a position that allows the center axis 40L to be interposed between the flush port 16 and the base 21 of the cover 11. More specifically, the flush port 16 is situated at a position that allows the center axis 40L to be interposed between the flush port 16 and an aperture 27 of the base 21 which will be mentioned later.

The flush port 16 is a through-hole that passes through the cover 11. The flush port 16 is a through-hole that is used when supplying a cleaning solution from outside of the housing 10 to the inner space of the housing 10. The flush port 16 passes through the upper surface 12 of the cover 11.

In the cover 11, the flush port 16 is situated on the positive side in the Z-direction with respect to the center of the cover 11. In other words, in the area of the cover 11, the flush port 16 is situated in an area that is closer to the shaft 40 than to the center of the cover 11. More specifically, on the upper surface 12, the flush port 16 is situated in an area that is closer to the shaft 40 than to the center of the upper surface 12 in the Z-direction.

When viewed in the Y-direction, the flush port 16 is situated such that an axis 16L of the flush port 16 extends through a position different from a position where the center axis 40L passes through. In other words, the flush port 16 is situated at a position different from the position through which the center axis 40L extends.

More specifically, when viewed in the Y-direction, the flush port 16 is situated away from the center axis 40L towards the negative side in the X-direction. The flush port 16 may be situated at a position away from the center axis 40L towards the positive side in the X-direction.

The axis 16L is an axis that passes through the center of the flush port 16, which is a through-hole. If the through-hole has a shape of a cylinder or a column, the center axis of such a cylinder or a column is the axis 16L.

As shown in FIG. 4, the base 21 is a member formed into a shape of a plate that forms an outer shape of the housing 10 along with the cover 11. The base 21 is a portion of the surgical instrument 1 that is attached to the surgical robot. On the base 21, the cover 11 is situated in the positive side in the Y-direction. The surface of the base 21 on the negative side in the Y-direction is the mounting surface 22. The mounting surface 22 is a surface for mounting the surgical instrument 1 on the surgical robot and is a surface that comes in contact with the surgical robot. The surgical robot is not illustrated in FIG. 4.

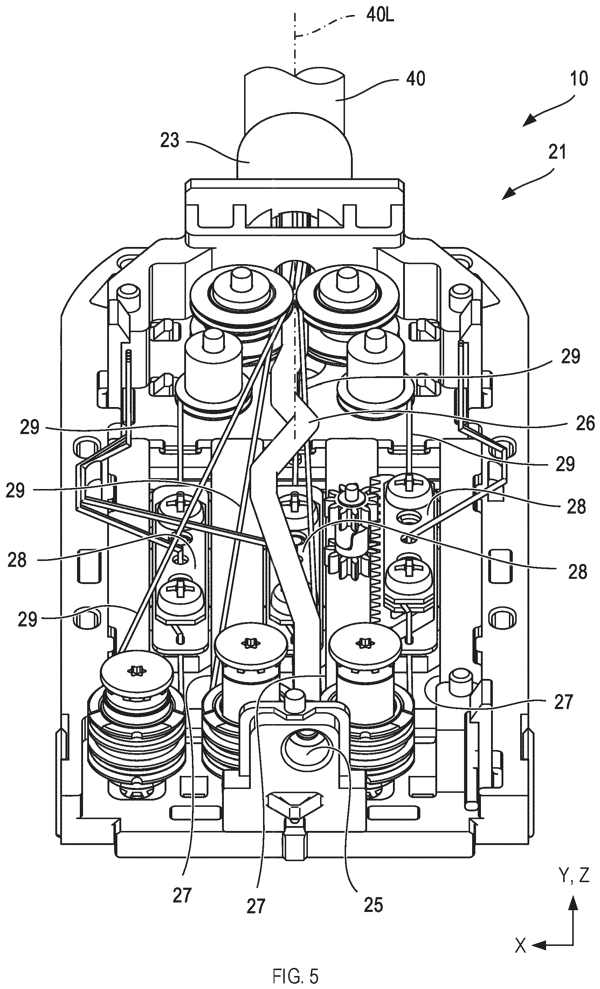

As shown in FIG. 1 and FIG. 5, for example, the base 21 includes a shaft mounting portion 23 for mounting the shaft 40 at one end of the base 21 situated on the positive side in the Z-direction. The shaft mounting portion 23 is disposed at a protrusion of the base 21 on the side of the cover 11 (in other words, on the positive side in the Y-direction). The shaft mounting portion 23 is disposed at the center of the base 21 in the X-direction. The shaft mounting portion 23 has a cylindrical shape protruding towards the positive side in the Z-direction. The shaft 40 is inserted into the shaft mounting portion 23 and retained in an inside of the shaft mounting portion 23.

A main flush port 25 is disposed at one end of the base 21 situated on the negative side in the Z-direction. The main flush port 25 is a through hole that is used to supply the cleaning solution from the outside of the housing 10 to the inside of the shaft 40.

On the other side of the shaft 40 on the base 21, the main flush port 25 is disposed at a portion of the base 21 extending towards the cover 11 side (in other words, on the positive side in the Y-direction). The main flush port 25 is a through hole extending in the Z-direction. The main flush port 25 may be situated at a position deviated from the center axis 40L of the shaft 40, or, the main flush port 25 may be situated at a position situated on the center axis 40L.

A tube 26 is coupled to the main flush port 25 (see FIG. 5). The tube 26 is a member that is used when supplying the cleaning solution to the inside of the shaft 40 along with the main flush port 25.

The tube 26 is a member formed into a cylindrical shape. The tube 26 is a member formed by using a flexible material such as a resin. The tube 26 is situated along the Z-direction. The tube 26 may be situated to have a linear shape, or may be situated to have a shape formed by combining straight lines and curved lines, or may be situated to have a curved shape.

The tube 26 is situated between the flush port 16 and the base 21. More specifically, the tube 26 is situated between the flush port 16 and an aperture 27 of the base 21 which will be explained later.

When viewed in the Y-direction, the tube 26 is situated such that the axis 16L of the flush port 16 passes and extends through a position different from a position where the center axis 40L passes through. More specifically, when viewed in the Y-direction, the tube 26 is situated at a position away from the flush port 16 towards the positive side in the X-direction. The tube 26 may be situated at a position away from the flush port 16 towards the negative side in the X-direction.

One end of the tube 26 situated on the negative side in the Z-direction is coupled to the main flush port 25. The other end of the tube 26 situated on the positive side in the Z-direction is situated in the inside of the shaft 40.

As shown in FIG. 4, the base 21 includes three apertures 27, 27, 27 and three sliders 28, 28, 28. Hereinafter, when an explanation is given about a single aperture 27, the other two apertures 27, 27 also have the same configuration. Hereinafter, the aperture 27 is also referred to as a driven groove. Hereinafter, when an explanation is given about a single slider 28, the other two sliders 28, 28 also have the same configuration.

The aperture 27 is a through hole that connects the inner space of the housing 10 to the outside. The aperture 27 is a through hole disposed on the base 21. The aperture 27 is an elongated hole extending in the Z-direction. The three apertures 27, 27, 27 are aligned at equal intervals in the X-direction.

In the Z-direction, the three apertures 27, 27, 27 are situated in a central area of the base 21. The flush port 16 is situated closer to the positive side in the Z-direction than the three apertures 27, 27, 27 are. In other words, the flush port 16 is situated in an area closer to the shaft 40 than the three apertures 27, 27, 27 are.

The number of the aperture 27 can be determined based on movements or the like of a joint and/or a forceps in the end effector 50. In other words, the number of the aperture 27 can be determined based on the movements made in accordance with the specification required in the surgical instrument 1. The number of the aperture 27 may be larger than three or smaller than three depending on the specification required.

The slider 28 is configured to receive a driving force transmitted from the surgical robot and to transmit the driving force to the end effector 50. The slider 28 is attachable to and detachable from the surgical robot.

The slider 28 is situated such that the slider 28 can move relative to the base 21. More specifically, the slider 28 is situated such that the slider 28 can move relative to the base 21 in a straight direction. The slider 28 may be situated such that the slider 28 can rotate relative to the base 21.

The sliders 28, 28, 28 are respectively situated in the three apertures 27, 27, 27. The sliders 28, 28, 28 are situated such that each of the sliders 28, 28, 28 can move in the inside of corresponding one of the apertures 27, 27, 27 in the Z-direction. In other words, the slider 28 is situated such that the slider 28 can move relative to the base 21 in a straight direction. All of the apertures 27, 27, 27 may have one of the sliders 28, 28, 28 situated therein, or only some of the apertures 27, 27, 27 may have one of the sliders 28, 28, 28 situated therein.

As shown in FIG. 5, the base 21 includes three wires 29, 29, 29. The wires 29, 29, 29 are configured to transmit the driving force transmitted to the slider 28 to the end effector 50. One or two wires 29, 29 are situated on the slider 28. Hereinafter, when an explanation is given about a single wire 29, the other two wires 29, 29 also have the same configuration.

The wire 29 is a wire formed into a long shape by using an electrically conductive material. The wire 29 may be formed by using a metallic material used in a manipulator of a surgical robot system, such as stainless steel, tungsten, an alloy containing tungsten, or a piano wire (for example, those specified in JIS G 3522).

As shown in FIG. 1, the shaft 40 is a member formed into a cylindrical shape that extends in the Z-direction from the base 21 of the housing 10. The shaft 40 may have a tubular shape other than the cylindrical shape.

The end effector 50 is situated in one end of the shaft 40 situated on the positive side in the Z-direction. As shown in FIG. 5, the other end of the shaft 40 situated on the negative side in the Z-direction is mounted to the shaft mounting portion 23 of the base 21. The inner space of the cylindrical shaft 40 communicates with the inner space of the housing 10.

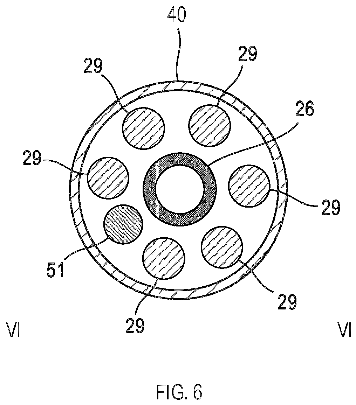

As shown in FIG. 6, the tube 26, the wires 29, and an electrical wiring 51 are situated in the inside of the shaft 40. The electrical wiring 51 is used to supply an electric current to the end effector 50.

The tube 26 is situated near the center in the inside of the shaft 40. The wires 29 and the electrical wiring 51 are situated around the tube 26. More specifically, six wires 29 and one electrical wiring 51 are situated around the tube 26.

The end effector 50 shown in FIG. 1 is an instrument used in a surgery. The end effector 50 has a function of a monopolar electric scalpel and a forceps. The end effector 50 may have a function of only one of the electric scalpel or the forceps, or may also have different functions.

As shown in FIG. 6, the six wires 29 and the electrical wiring 51 situated through the inside of the shaft 40 are coupled to the end effector 50. The end effector 50 is configured such that the six wires 29 open and close the forceps and change the orientation of the forceps. The end effector 50 is configured such that a high frequency current supplied by the electrical wiring 51 is transmitted to the forceps.

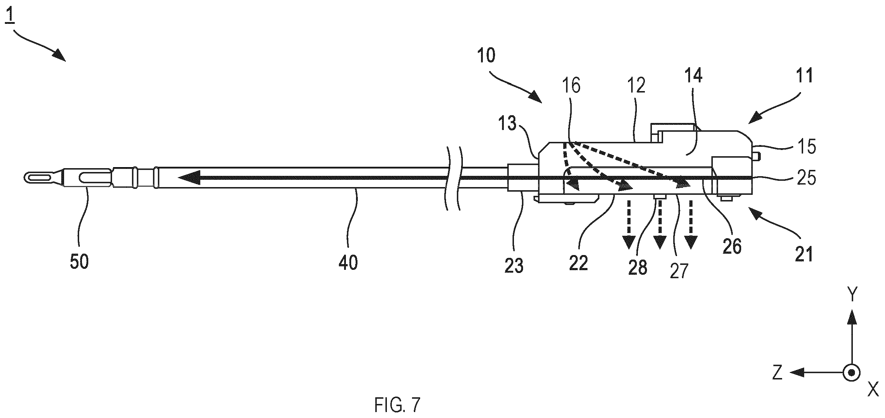

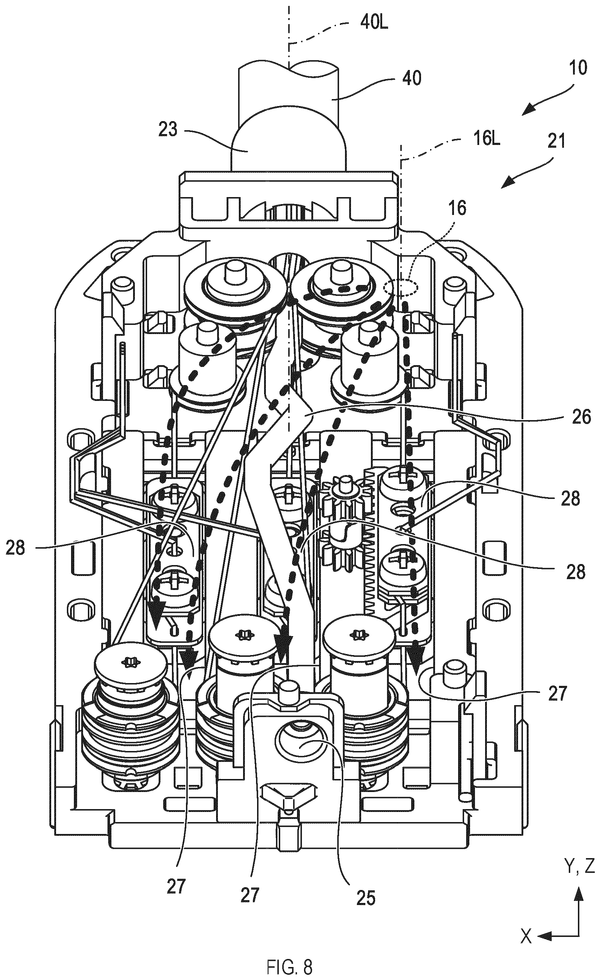

Next, a flow of the cleaning solution when cleaning the surgical instrument 1 configured as mentioned above will be explained with reference to FIG. 7 and FIG. 8. Firstly, a flow of the cleaning solution supplied from the flush port 16 will be explained.

As shown in FIG. 7, the cleaning solution supplied from the flush port 16 flows into the inner space of the housing 10 from the outside. At this time, the cleaning solution flows from the positive side to the negative side in the Y-direction. The cleaning solution spreads and flows in the inner space of the housing 10. The cleaning solution removes contaminants and the like in the inner space of the housing 10. In other words, the cleaning solution cleans the contaminants and the like in the inner space of the housing 10.

The cleaning solution that flowed into the inner space of the housing 10 then flows through the inside of the housing 10 towards a position where the housing 10 communicates with the outside. More specifically, the cleaning solution flows towards the apertures 27, 27, 27, a connection between the cover 11 and the base 21, and the like.

When comparing the apertures 27, 27, 27 with the connection between the cover 11 and the base 21, a difference is found in the ease of flow of the cleaning solution. In other words, the ease of flow of the cleaning solution from the apertures 27, 27, 27 to the outside of the housing 10 is different from the ease of flow of the cleaning solution from the aforementioned connection to the outside of the housing 10.

A flow passage resistance at a portion of the apertures 27, 27, 27 where the cleaning solution flows is smaller than the flow passage resistance at the connection. Thus, the cleaning solution is likely to flow towards the apertures 27, 27, 27 after spreading in the inner space of the housing 10. The cleaning solution is less likely to flow in other directions.

As shown in FIG. 7 and FIG. 8, the cleaning solution that flowed into the inner space of the housing 10 flows from the flush port 16 towards the negative side in the Y-direction and is likely to flow towards the negative side in the Z-direction. In other words, the cleaning solution that flowed into the inner space of the housing 10 is less likely to flow towards the positive side in the Z-direction.

In the Z-direction, the shaft 40 is situated in the positive side with reference to the flush port 16. Thus, the cleaning solution that flowed into the inner space of the housing 10 is less likely to flow towards the shaft 40, and therefore, less likely to flow into the inside of the shaft 40.

A part of the cleaning solution that flowed into the inner space of the housing 10 is also likely to flow towards the positive side in the X-direction. The shaft 40 extends in the Z-direction. A flow towards the positive side in the X-direction is a flow in a direction crossing the shaft 40, and thus is not a flow towards the inside of the shaft 40. Thus, the cleaning solution that has flowed into the inner space of the housing 10 is less likely to flow towards the inside of the shaft 40, and therefore, less likely to flow into the inside of the shaft 40.

The cleaning solution flows from the flush port 16 into the inner space of the housing 10 without colliding with the tube 26 without colliding with other elements. Hereinafter, the event that the cleaning solution collides with the tube without colliding with other elements is also referred to as "direct collision". Accordingly, it is less likely to happen that the flow direction of the cleaning solution is changed to the direction towards the inside of the shaft 40 due to the direct collision with the tube 26.

Next, a flow of the cleaning solution that is supplied from the main flush port 25 will be explained. As shown in FIG. 7 and FIG. 8, the cleaning solution supplied from the main flush port 25 flows into the tube 26. The cleaning solution is guided by the tube 26 to the inside of the shaft 40.

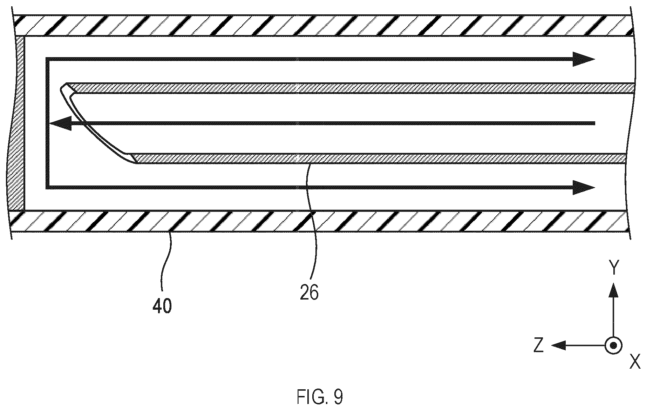

As shown in FIG. 9, the cleaning solution that was guided by the tube 26 flows to the inside of the shaft 40. An end of the shaft 40 on the positive side in the Z-direction is closed. The cleaning solution that flowed out from the tube 26 towards the positive direction in the Z-direction therefore changes its direction of flow to the negative direction in the Z-direction in the inside of the shaft 40.

The cleaning solution that changed its direction of flow removes the contaminants and the like that exist in the inside of the shaft 40. In other words, the cleaning solution cleans the inside of the shaft 40. The cleaning solution that returned to the inner space of the housing 10 flows out to the outside of the housing 10 from the apertures 27, 27, 27 and the like.

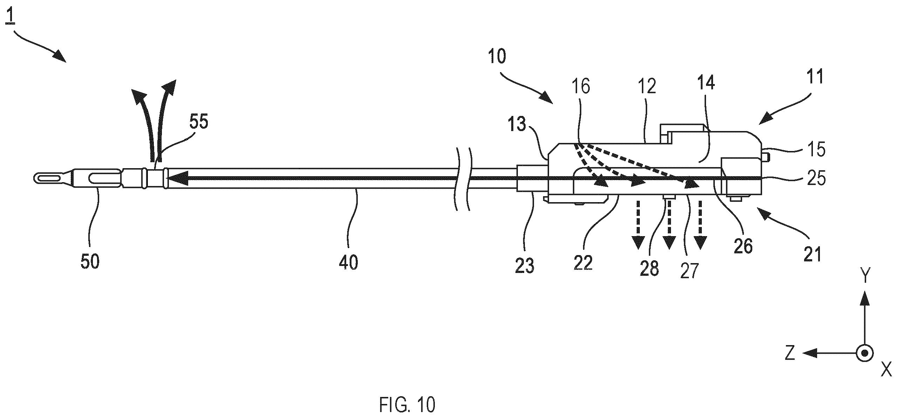

As shown in FIG. 10, the shaft 40 may include a discharge hole 55 that discharges the cleaning solution to the outside. In FIG. 10, the discharge hole 55 is disposed at a position in the shaft 40 where the end effector 50 is mounted. The discharge hole 55 may be disposed at a position other than where the end effector 50 is mounted.

According to the surgical instrument 1 configured as above, the cleaning solution that flowed from the flush port 16 into the inner space of the housing 10 crosses the center axis 40L of the shaft 40 and is likely to flow from the apertures 27, 27, 27 to the outside of the housing 10. In other words, the main direction of the flow of the cleaning solution is likely to be the direction towards the apertures 27, 27, 27. The direction of the flow of the cleaning solution is likely to be the direction crossing the center axis 40L of the shaft 40.

Among components of the direction of the flow of the cleaning solution, a component of the flow towards the inside of the shaft 40 is likely to be smaller than a component of the flow towards the apertures 27, 27, 27. In other words, the direction of the flow of the cleaning solution is less likely to be directed from the inner space of the housing 10 towards the inside of the shaft 40.

The cleaning solution supplied from the flush port 16 to the inside of the housing 10 is likely to be directed towards the center of the housing 10. In other words, among the components of the direction of the flow of the cleaning solution directed from the flush port 16 towards the apertures 27, 27, 27, components of the negative direction of the Z-axis is likely to increase. For example, the cleaning solution is likely to flow towards the direction away from the shaft 40. Thus, the cleaning solution is less likely to flow from the inner space of the housing 10 to the inside of the shaft 40.

Since the axis 16L of the flush port 16 extends by passing through a position different from a position where the center axis 40L of the shaft 40 passes through, the main direction of the flow of the cleaning solution that enters from the flush port 16 along the axis 16L is likely to be a direction different from the direction towards the inside of the shaft 40. Thus, the cleaning solution is less likely to flow from the inner space of the housing 10 to the inside of the shaft 40.

Accordingly, a need for directing the cleaning solution after cleaning the inner space of the housing 10, which has flowed into the inside of the shaft 40, back from the inside of the shaft 40 to the inner space of the housing 10 to have the cleaning solution flow to the outside of the housing 10 decreases. As a result, the duration of time required for cleaning the surgical instrument 1 is less likely to be prolonged, and thus, a deterioration of cleaning efficiency is likely to be inhibited.

## Claims

1. A surgical instrument (1) comprising:
a housing (10) including an inner space; and
a shaft (40) formed into a cylindrical shape extending from the housing (10) and communicating with the inner space,
the housing (10) comprising a cover (11) and a base (21) that is a plate member,
the housing (10) comprising a flush port (16) that is a through hole for a cleaning solution to enter from outside, and at least one aperture (27) from which the cleaning solution flows to the outside,
the flush port (16) being disposed on the cover (11), and the at least one aperture (27) being situated in the base (21),
the flush port (16) being situated in an area of the housing (10) closer to the shaft (40) than the at least one aperture (27) is,
the flush port (16) being situated at a position that allows a center axis (40L) of the shaft (40) to be interposed between the flush port (16), and the at least one aperture (27) and, when the cover (11) is viewed in a Y-direction, being situated away from the center axis (40L) of the shaft (40) in an X-direction,
the X-direction and the Y-direction extending along a plane situated perpendicular to the center axis (40L) and being perpendicular to each other, and
the X-direction extending along the base (21).

2. The surgical instrument (1) according to claim 1,
wherein the flush port (16) is situated such that an axis (16L) of the flush port (16) passes and extends through a position different from a position where the center axis (40L) of the shaft passes through.

3. The surgical instrument (1) according to claim 1 or 2,
wherein the flush port (16) is situated in an area of the housing (10) closer to the shaft (40) than to the center of the housing (10) in a direction in which the shaft (40) extends.

## Patentansprüche

1. Chirurgisches Instrument (1), enthaltend:
ein Gehäuse (10), das einen inneren Raum einschließt; und
einen Schaft (40), der in einer zylindrischen Form gebildet ist, die sich vom Gehäuse erstreckt (10) und mit dem inneren Raum in Verbindung steht,
wobei das Gehäuse (10) eine Abdeckung (11) und eine Basis (21) enthält, die ein Plattenelement ist,
wobei das Gehäuse (10) einen Flush-Port (16), der ein Durchgangsloch für eine Reinigungslösung ist, um von außen einzutreten, und mindestens eine Öffnung (27) enthält, von dem die Reinigungslösung nach außen fließt,
wobei der Flush Port (16) an der Abdeckung (11) angeordnet ist, und die mindestens eine Öffnung (27) in der Basis (21) positioniert ist,
wobei der Flush-Port (16) in einem Bereich des Gehäuses (10) näher am Schaft (40) positioniert ist als die mindestens eine Öffnung (27) ist,
wobei der Flush-Port (16) an einer Position positioniert ist, die einer Mittelachse (40L) des Schafts (40) erlaubt, zwischen dem Flush-Port (16) und der mindestens einen Öffnung (27) eingeschoben zu sein, und, wenn die Abdeckung (11) in Y-Richtung betrachtet wird, entfernt von der Mittelachse (40L) des Schafts (40) in X-Richtung positioniert ist,
wobei sich die X-Richtung und die Y-Richtung entlang einer Ebene erstrecken, die senkrecht zu der Mittelachse (40L) positioniert ist, und senkrecht zueinander stehen, und
wobei sich die X-Richtung entlang der Basis (21) erstreckt.

2. Chirurgisches Instrument (1) gemäß Anspruch 1,
wobei der Flush-Port (16) so positioniert ist, dass eine Achse (16L) des Flush-Ports (16) durch eine Position verläuft und sich erstreckt, die sich von einer Position unterscheidet, an der die Mittelachse (40L) des Schafts hindurch verläuft.

3. Chirurgisches Instrument (1) gemäß Anspruch 1 oder 2,
wobei der Flush-Port (16) in einem Bereich des Gehäuses (10) näher am Schaft (40) als zu der Mitte des Gehäuses (10) in eine Richtung positioniert ist, in die sich der Schaft (40) erstreckt.

## Revendications

1. Un instrument chirurgical (1) comprenant :
un boîtier (10) incluant un espace intérieur; et
une tige (40) formée en une forme cylindrique s'étendant depuis le boîtier (10) et communiquant avec l'espace intérieur,
le boîtier (10) comprenant un couvercle (11) et une base (21) qui est un membre de plaque,
le boîtier (10) comprenant un port de rinçage (16) qui est un trou traversant pour une solution de nettoyage pour entrer de l'extérieur, et au moins une ouverture (27) de laquelle la solution nettoyante s'écoule vers l'extérieur,
le port de rinçage (16) étant disposé sur le couvercle (11), et l'au moins une ouverture (27) étant située dans la base (21),
le port de rinçage (16) étant situé dans une zone du boîtier (10) plus près de la tige (40) que l'au moins une ouverture (27) est,
le port de rinçage (16) étant situé à une position qui permet un axe central (40L) de la tige (40) à être interposé entre le port de rinçage (16) et l'au moins une ouverture (27) et, lorsque le couvercle (11) est vu dans une direction Y, étant situé à l'écart de la axe central (40L) de la tige (40) dans une direction X,
la direction X et la direction Y s'étendant le long d'un plan situé perpendiculairement à la axe central (40L) et étant perpendiculaires l'un à l'autre, et
la direction X s'étendant le long de la base (21).

2. L'instrument chirurgical (1) selon la revendication 1,
dans lequel le port de rinçage (16) est situé de manière à ce qu'un axe (16L) du port de rinçage (16) passe et s'étend à travers une position différente d'une position dans laquelle l'axe central (40L) de la tige passe à travers.

3. L'instrument chirurgical (1) selon la revendication 1 ou 2,
dans lequel le port de rinçage (16) est situé dans une zone du boîtier (10) plus près de la tige (40) qu'au centre du boîtier (10) dans une direction dans laquelle s'étend la tige (40).
